# EUROPÄISCHE PATENTSCHRIFT

(11) **EP 3 305 327 B1**
(45) Veröffentlichungstag und Bekanntmachung des Hinweises auf die Patenterteilung: **20.02.2019**
(21) Anmeldenummer: 17194453.1
(22) Anmeldetag: 02.10.2017
(51) Int. Cl.: A61K 45/06, A61K 31/122, A61K 31/14, A61K 31/205, A61K 31/4415, A61K 31/455, A61K 31/519, A61K 31/525, A61K 31/593, A61K 31/714, A61P 3/06

(54) **LIPIDSENKER**
LIPID LOWERING DRUG
HYPOLIPIDÉMIANT

(30) Priorität: 06.10.2016 AT 502022016
(43) Veröffentlichungstag der Anmeldung: 11.04.2018
(73) Patentinhaber: Bioenergy Healthcare GmbH, 50999 Köln (DE)
(72) Erfinder: ROMBACH, Reinhold, 50999 Köln (DE); HERGERT, Jochen, 79379 Müllheim (DE)
(74) Vertreter: Diehl & Partner GbR

(56) Entgegenhaltungen:
- US-A- 6 054 128
- US-A1- 2006 009 486
- OLSZEWSKI A J ET AL: "REDUCTION OF PLASMA LIPID AND HOMOCYSTEINE LEVELS BY PYRIDOXINE FOLATE COBALAMIN CHOLINE RIBOFLAVIN AND TROXERUTIN IN ATHEROSCLEROSIS", ATHEROSCLER, ELSEVIER, AMSTERDAM, NL, Bd. 75, Nr. 1, 1. Januar 1989 (1989-01-01) , Seiten 1-6, XP008150797, ISSN: 0021-9150, DOI: 10.1016/0021-9150(89)90200-1
- DATABASE MEDLINE [Online] US NATIONAL LIBRARY OF MEDICINE (NLM), BETHESDA, MD, US; März 2006 (2006-03), GARCÉS P ANTONIO ET AL: "[Lowering plasma homocysteine with vitamins B6, B12, and folic acid. Effect on lipids concentration in patients with secondary hyperlipoproteinemia type IV, with and without Lovastatina treatment].", XP002776542, Database accession no. NLM16786732 & ARCHIVOS LATINOAMERICANOS DE NUTRICION MAR 2006, Bd. 56, Nr. 1, März 2006 (2006-03), Seiten 36-42, ISSN: 0004-0622
- MACMAHON M ET AL: "A pilot study with simvastatin and folic acid/vitamin B12 in preparation for the Study of the Effectiveness of Additional Reductions in Cholesterol and Homocysteine (SEARCH)", NMCD. NUTRITION METABOLISM AND CARDIOVASCULAR DISE, MILAN, IT, Bd. 10, Nr. 4, 1. August 2000 (2000-08-01) , Seiten 195-203, XP009104484, ISSN: 0939-4753

## Beschreibung

Die vorliegende Erfindung betrifft eine pharmazeutische Zusammensetzung zur therapeutischen Regulation des Lipidstoffwechsels, und insbesondere zur Behandlung oder Prävention einer Dyslipidämie, Dyslipoproteinämie oder Hypertriglyceridämie, enthaltend Folsäure, Vitamin B12 und Vitamin B6 gemäß den Ansprüchen. Offenbart ist die Verwendung einer entsprechenden Wirkstoffkombination zur Herstellung eines Medikaments, einer bilanzierten Diät oder von Nahrungsergänzungsmitteln.

Garces *et al.* berichten in ihrem Artikel *"*Lowering plasma homocysteine..." in den Archivos Latinoamericanos de Nutricion, Band 56, Nr. 1, März 2006, Seiten 36 bis 42, von der Verabreichung einer Vitaminzusammensetzung mit 10 mg/d Folsäure, 300 mg/d Vitamin B6 und 250 µg/d Vitamin B12 an Patienten mit Hyperhomocysteinämie und sekundärer Lipoproteinämie Typ IV nach einem Herzinfarkt.

Die Dokumente US 6,054,128 A und US 2006/0009486 A1 offenbaren Zusammensetzungen, die unter anderem je 400 µg Folsäure und Vitamin B12, sowie 100 mg Vitamin B6 bzw. 1 mg Folsäure, 400 µg Vitamin B12 und 25 mg Vitamin B6 sowie mehrfach ungesättigte Fettsäuren enthalten. Weiters enthalten diese Zusammensetzungen entweder 100 mg Weißdorn- und 150 mg Malabar-Tamarindenextrakt bzw. 20 mg Policosanol zur Reduzierung von Serum-Lipiden.

Es ist bekannt, zum Beispiel aus dem Dokument WO 98/50028 A1, Lipid-Plasmaspiegel, die erheblich über den Normalwerten liegen, mit der Gabe von fettlöslichen Vitaminen (Vitamine A, D, E, K) und MTP-Inhibitoren zu regulieren. Allerdings ist die Wirksamkeit, Sicherheit und Nachhaltigkeit dieser Behandlung umstritten.
Die vorliegende Erfindung schlägt stattdessen eine Zusammensetzung aus Folsäure, Vitamin B12 und B6 in relativ (nämlich in Bezug auf allgemeine Richtwerte) hoher Dosierung zur Anwendung in einem Verfahren der Regulation des Lipidstoffwechsels gemäß Anspruch 1 vor. Ein MTP-Inhibitor ist nicht nötig.

Gegenstände, die nicht durch den Schutzumfang der Ansprüche umfasst sind, sind nicht Teil der beanspruchten Erfindung.

Es wird vermutet, dass die Wirkung auf folgenden Mechanismen beruht: Vitamin B12 ist erforderlich, um eine 1-Kohlenstoffeinheit von Folsäure auf Homocystein zu übertragen und dann diese Einheit dem Lipid-Metabolismus zuzuführen. Vitamin B6 ist an einem Stoffwechselabzweig für die Remethylierung beteiligt und wirkt indirekt auf den Lipidstoffwechsel.
Die in dieser Anmeldung angegebenen Mengenangaben beziehen sich auf Gewichtsmengen der Wirkstoffe Folsäure, Vitamin B6 und Vitamin B12, so dass für Salze und Derivate erforderlichenfalls eine entsprechende Umrechnung zu erfolgen hat. Dies gilt analog für die in der vorliegenden Beschreibung angegebenen Wirkstoffanteile. Alternativ kann man die Verhältnisse auch auf molare Mengen beziehen, so dass unter der Annahme, dass 1 Mol des betreffenden Derivats oder Salzes 1 Mol Folsäure, Vitamin B6 oder Vitamin B12 enthält, die Molmengenverhältnisse für Folsäure, Vitamin B6, Vitamin B12 und deren Derivate und/oder Salze einheitlich ausgedrückt werden können.
"Folsäure" bezeichnet erfindungsgemäß N-Pteroylglutaminsäure der Formel I, 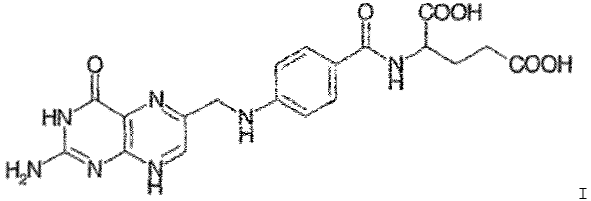 die unter diese Formel fallenden optischen Isomere sowohl als Gemische, z. B. als Racemat, als auch in Reinform, z. B. R- oder S-Enantiomere, eingeschlossen. Bevorzugt ist N-Pteroyl-L-glutaminsäure der Formel Ia:

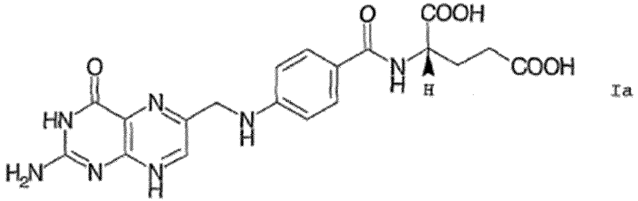

Zu den Folsäure-Derivaten gehören vor allem Folsäure-Metabolite sowie Amide und Ester der Folsäure wie auch der Metabolite. Vorteilhaft sind Amide und Ester, die unter physiologischen Bedingungen hydrolysierbar sind, wie Amide mit C₁-C₁₀-Alkylaminen oder Ester mit C₁-C₁₀-Alkoholen. Eine besondere Form der Amide sind N-Pteroylpolyglutaminsäuren.

Zu den Folsäure-Metaboliten gehören vor allem H₄-Folsäuren der Formel Ib 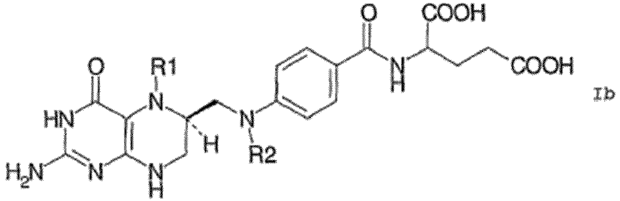 worin R1 für Wasserstoff, Methyl, -HC=O (Formyl) oder -HC=NH (Formimino) steht und R2 für Wasserstoff oder -HC=O (Formyl) steht, oder R1 und R2 zusammen genommen eine Methylen- oder Methenylbrücke bilden. Die unter diese Formel fallenden optischen Isomere sind obigen Ausführungen entsprechend eingeschlossen, wobei auch hier die L-Glutaminsäure-Derivate bevorzugt sind. Insbesondere sind Tetrahydrofolsäure, 5-Methyltetrahydrofolsäure, 5,10-Methylentetrahydrofolsäure, 5-Formyltetrahydrofolsäure, 10-Formyltetrahydrofolsäure, 5-Formiminotetrahydrofolsäure und 5,10-Methenyltetrahydrofolsäure zu nennen.

Zu physiologisch akzeptablen Salzen von Folsäure bzw. Folsäure-Derivaten gehören Säure- und Basenadditionssalze sowie entsprechende Mischformen.

Zu den Säureadditionssalzen zählen Salze von Folsäure bzw. Folsäure-Derivaten mit anorganischen Säuren, wie Salzsäure, Schwefelsäure, Salpetersäure oder Phosphorsäure, oder organischen Säuren, insbesondere Carbonsäuren, z. B. Essigsäure, Weinsäure, Milchsäure, Zitronensäure, Apfelsäure, Mandelsäure, Ascorbinsäure, Maleinsäure, Fumarsaure, Gluconsäure oder Sulfonsäuren, z. B. Methansulfonsäure, Benzolsulfonsäure und Toluolsulfonsäure.

Zu den Basenadditionssalzen zählen Salze von Folsäure bzw. FoIsäure-Derivaten mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z. B. Methylamin, Dimethylamin, Trimethylamin, Triethylamin, Ethylamin, Diethylamin, Ethylendiamin, Ethanolamin, Diethanolamin, 1-Amino-2-propanol, 3-Amino-1-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt sind Salze mit anorganischen Basen, z. B. Na-, K-, Mg-, Ca-, Zn-, Cr- und Fe-Folate.

"Vitamin B6" bezeichnet erfindungsgemäß 4,5-Bis(hydroxymethyl)-2-methyl-3-pyridinol der Formel II 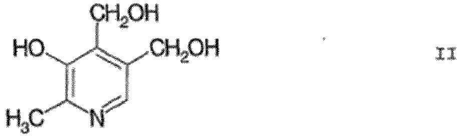 auch als Pyridoxin (INN) bezeichnet.

Zu den Vitamin B6-Derivaten gehören vor allem Pyridoxale und Pyridoxamine sowie Ester der Pyridoxine, Pyridoxale und Pyridoxamine. Vorteilhaft sind auch hier Ester, die unter physiologischen Bedingungen hydrolysierbar sind. Insbesondere zu nennen sind in diesem Zusammenhang die Pyridoxine, Pyridoxale und Pyridoxamine der Formel IIa: 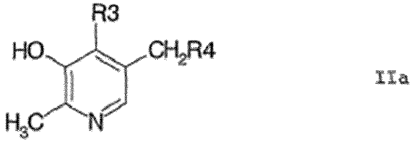 worin R3 für CH₂OH, CHO oder CH₂NH₂ steht und R4 für OH oder OPO₃H₂ steht.

Zu physiologisch akzeptablen Salzen von Vitamin B6 bzw. Vitamin B6-Derivaten gehören insbesondere Säureadditionssalze, z. B. mit den oben genannten anorganischen und organischen Säuren. Insbesondere ist das Hydrochlorid, vor allem Pyridoxin-HCl, zu nennen.

"Vitamin B12" wird auch als Cyanocobalamin oder Cobalamin bezeichnet, Formel III: 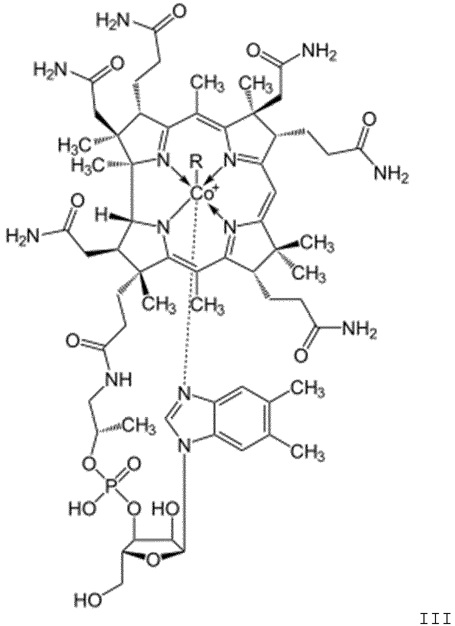 wobei R eine Cyanogruppe repräsentiert. Zu den Vitamin-B 12-Derivaten gehören insbesondere Cobalamine, in denen die Cyanogruppe ("R") des Cyanocobalamins durch andere Koordinationspartner des Cobalts ersetzt ist. Hierzu zählen vor allem das Aquocobalamin ("Vitamin B12a"), Hydroxocobalamin ("Vitamin B12b"), Nitrosocobalamin, Methylcobalamin ("Vitamin MeB12") und Adenosylcobalamin (Coenzym B12). Zu den physiologisch akzeptablen Salzen von Vitamin B12 bzw. Vitamin B12-Derivaten gehören insbesondere Säureadditionssalze z. B. mit den oben genannten anorganischen und organischen Säuren. Insbesondere ist das Acetat des Hydroxocobalamins zu nennen.

Folsäuren, B6- und B12-Vitamine sind hinlänglich bekannt und können entweder bezogen oder in an sich bekannter Weise zur Verfügung gestellt werden.

Neben den Folsäure-, Vitamin B6- und Vitamin-B 12-Komponenten kann die erfindungsgemäße Zusammensetzung weitere Wirkstoffe enthalten. Bei diesen Wirkstoffen kann es sich insbesondere um solche handeln, deren Wirkung der Folsäure-, Vitamin B6- bzw. Vitamin B12-vermittelten Wirkung ähnlich ist oder diese ergänzt und die insbesondere den erfindungsgemäßen Anwendungszwecken entspricht. Hervorzuheben sind Cholin (Tagesdosis mindestens 10 mg, z. B. 90-500 mg), Traubenkernextrakt (Tagesdosis mindestens 10 mg, z. B. 40-500 mg) und/oder Artischockenextrakt (Tagesdosis mindestens 10 mg, z. B. 40-500 mg). Auch Carotinoide, insbesondere Xanthophylle, weiter insbesondere Astaxanthin können zusätzlich verabreicht werden. Ferner können weitere Vitamine wie Vitamin A, Vitamin C, Vitamin D und/oder Vitamin E zugefügt werden. Insbesondere können Vitamin B2 (Riboflavin), Vitamin B3 (Niacin), Coenzym Q₁₀ und Vitamin D3 in der Zusammensetzung enthalten sein. Geeignete Tagesdosen liegen bei Vitamin B2 im Bereich 0,5 mg bis 40 mg, bei Vitamin B3 im Bereich 5 mg bis 30 mg, bei Coenzym Q₁₀ im Bereich von 10 bis 200 mg, bei Vitamin D3 im Bereich 5 µg bis 50 µg. Darüber hinaus können Betain und/oder Omega-3-Fettsäuren enthalten sein.

Die Behandlung mit der erfindungsgemäßen Zusammensetzung ist insbesondere dann angezeigt, wenn der HDL-Wert erniedrigt und/oder der LDL-Wert und/oder das LDL/HDL-Verhältnis erhöht ist; jedoch auch, wenn Cholesterin allein, Triglyceride allein oder sowohl Cholesterin wie auch Triglyceride erhöht sind. Unter einer Erhöhung des LDL/HDL-Verhältnisses wird im Rahmen dieser Anmeldung ein LDL/HDL-Verhältnis von über 2,5, insbesondere von über 3,0, von über 3,5 oder gar von über 4,0 verstanden. Die erfindungsgemäßen Zusammensetzungen gewinnen gemeinhin bei Erwachsenen mit zunehmendem Lebensalter an Bedeutung.
In einer 3monatigen Studie mit ca. 350 Probanden sank bei Verabreichung einer Tagesdosis von 1 mg Folsäure, 1 mg Vitamin B12 und 50 mg Vitamin B6 der Anteil derjenigen Probanden, deren LDL-Wert über dem Referenzwert lag, von über 36 % auf unter 22 %. Ferner sanken im Mittel der Cholesterin- und der Triglycerid-Wert, während der HDL-Wert stieg. Die Veränderungen waren umso größer, je größer die Ausgangsabweichung vom jeweiligen Referenzwert war.
Erfindungsgemäß wird dem zu behandelnden Individuum eine wirksame Menge der erfindungsgemäßen Wirkstoffkombination aus Folsäure-Komponente, Vitamin B12-Komponente und Vitamin B6-Komponente, in der Regel der pharmazeutischen oder lebensmitteltechnologischen Praxis entsprechend formuliert, oral verabreicht. Wirksam ist eine Menge erfindungsgemäß insbesondere dann, wenn sie eine signifikante Senkung des Blutfettspiegels, vorteilhafterweise bis in den Normalbereich, bewirkt. Von besonderer Bedeutung ist eine Verringerung des LDL/HDL-Verhältnisses bis unter 4,0, unter 3,5, unter 3,0 oder unter 2,5.
Die Behandlung erfolgt in der Regel durch einmaliges oder mehrmaliges tägliches, oder intermittierendes (d. h. mit einem oder mehreren Tagen bis zu einer Woche Abstand) Verabreichen einer geeigneten Dosis gegebenenfalls zusammen oder im Wechsel mit anderen Wirkstoffen oder wirkstoffhaltigen Präparaten, so dass einem zu behandelnden Individuum mit dem Gewicht eines durchschnittlichen Erwachsenen von etwa 75 kg in der Regel eine Tagesmindestdosis von etwa 0,2 mg, vorzugsweise etwa 0,5 mg und besonders vorteilhafterweise etwa 0,6 mg Folsäure, von etwa 4 mg, vorzugsweise etwa 10 mg und besonders vorteilhafterweise etwa 20 mg Vitamin B6 (wenn vorhanden), sowie von etwa 8 µg, vorzugsweise etwa 0,1 mg und besonders vorteilhafterweise etwa 0,2 mg Vitamin B12 verabreicht wird. Andererseits beträgt die Tageshöchstdosis in der Regel etwa 10 mg, vorzugsweise etwa 1,1 mg und besonders bevorzugt etwa 0,9 mg Folsäure, etwa 100 mg, vorzugsweise etwa 75 mg und vorteilhafterweise etwa 50 mg Vitamin B6, sowie etwa 1,2 mg, vorzugsweise etwa 0,8 mg und besonders bevorzugt etwa 0,5 mg Vitamin B12.

Die Behandlung erfolgt in der Regel über einen angemessenen Zeitraum im Bereich von Wochen oder Monaten. Zweckmäßig ist eine Normalisierung der Lipidspiegel innerhalb eines Behandlungszeitraums von etwa 1 bis 6 Monaten. Erforderlichenfalls wird die Behandlung auch nach Normalisierung der Lipidspiegel fortgesetzt.

Zu den pharmazeutischen Zusammensetzungen gehören insbesondere Nahrungsergänzungsmittel und funktionale oder diätetische Lebensmittel. Die erfindungsgemäßen Lebensmittel besitzen neben einer vorwiegend nährwertbezogenen Funktion zusätzlich eine wirkstoffbezogene Funktion. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen Wirkstoffkombination, wobei die nährwertbezogene Funktion des Nahrungsergänzungsmittels für sich genommen in den Hintergrund tritt.

Einem Aspekt zufolge betrifft die vorliegende Erfindung Formulierungen, enthaltend
i) wenigstens einen Wirkstoff aus der Folsäure-Gruppe (Folsäure, physiologisch akzeptable Derivate und/oder Salze davon),
ii) wenigstens einen Wirkstoff aus der Vitamin B12-Gruppe (Vitamin B12, physiologisch akzeptable Derivate und/oder Salze davon), und optional iii) wenigstens einen Wirkstoff aus der Vitamin B6-Gruppe (Vitamin B6, physiologisch akzeptable Derivate und/oder Salze davon), sowie gegebenenfalls wenigstens einen weiteren Wirkstoff und eine Formulierungsgrundlage, in den erfindungsgemäß angegebenen Mengen-Verhältnissen.
Somit umfasst die Wirkstoffkombination im Sinne der Erfindung als Wirkstoffkomponente i) Folsäure, ein physiologisch akzeptables Derivat und/oder Salz davon. Gemische dieser Formen sind möglich. Gemäß einer besonderen Ausführungsform besteht die Wirkstoffkomponente i) aus wenigstens 90 Gew.-% Folsäure.
Weiterhin umfasst die Wirkstoffkombination im Sinne der Erfindung als Wirkstoffkomponente ii) Vitamin B12, ein physiologisch akzeptables Derivat und/oder Salz davon. Gemische dieser Formen sind ebenfalls möglich. Gemäß einer besonderen Ausführungsform besteht die Wirkstoffkomponente ii) aus wenigstens 90 Gew.-% Cobalamin.

Weiterhin umfasst die Wirkstoffkombination im Sinne der Erfindung als Wirkstoffkomponente iii) Vitamin B6, ein physiologisch akzeptables Derivat und/oder Salz davon. Gemische dieser Formen sind ebenfalls möglich. Gemäß einer besonderen Ausführungsform besteht die Wirkstoffkomponente iii) aus wenigstens 90 Gew.-% Pyridoxin-HCl.

Der Anteil der Wirkstoffkombination an der Formulierung ist größer als ein gegebenenfalls in natürlichen Quellen, insbesondere Lebensmitteln, vorhandener Anteil. In diesem Sinne sind die erfindungsgemäßen Zusammensetzungen im Hinblick auf die Wirkstoffkombination angereichert. Der Anteil der Wirkstoffkombination aus i), ii) und iii) an der Formulierung beträgt vorzugsweise mindestens etwa 0,01 Gew.-%, vorteilhafterweise mindestens etwa 0,05 Gew.-% und insbesondere mindestens etwa 0,1 Gew.-%. Im Falle einer pharmazeutischen Zusammensetzung liegt der Anteil in der Regel bei etwa 1 bis 60 Gew.-%, vorzugsweise bei etwa 5 bis 35 Gew.-% und insbesondere bei etwa 10 bis 30 Gew.-%, im Falle eines Nahrungsergänzungsmittels und vor allem bei Lebensmitteln gegebenenfalls entsprechend niedriger, wenn die Formulierung in größeren Mengen zugeführt wird. Vorzugsweise enthalten die Formulierungen die angegebene Tagesdosis.

Angaben in Gew.-% beziehen sich auf das Gesamtgewicht der Formulierung.

Die Formulierungsgrundlage erfindungsgemäßer pharmazeutischer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten, und auch andere Hilfsstoffe, deren Eigenschaften einer physiologischen Anwendung nicht entgegenstehen. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger ; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H. P., Lexikon der Hilfsstoffe für Pharmazie, Kosmetik und angrenzende Gebiete, 5. Auflage, ECV·Editio Cantor Verlag, Aulendorf 2002, dargestellt.

Nahrungskomponenten enthalten in der Regel eine oder mehrere Aminosäuren, Kohlenhydrate oder Fette und sind für die menschliche Ernährung geeignet. Sie umfassen Einzelkomponenten, häufig pflanzliche aber auch tierische Produkte, insbesondere Zucker gegebenenfalls in Form von Sirups, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker.

Zu den essentiellen Nährstoffen zählen insbesondere Vitamine, Provitamine, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Arginin, Histidin, Cystein und Tyrosin. Als Spurenelemente seien genannt: essentielle Spurenelemente, deren Notwendigkeit für den Menschen erwiesen ist und deren Mangel zur Manifestation klinischer Symptome führt: Eisen, Kupfer, Zink, Chrom, Selen, Calcium, Magnesium, Kalium, Lithium, Kobalt, Molybdän, Iod, Silicium, Fluor. Ebenso Elemente, deren Funktion für den Menschen noch nicht genügend gesichert ist: Zinn, Nickel, Vanadium, Mangan. Als für den Menschen essentielle Fettsäuren seien genannt: Linolsäure und die Omega-3-Fettsäuren Linolensäure, Eicosapentaensäure (EPA) und Docosahexaensäure (DHA). Eine umfassende Aufzählung von Vitaminen findet sich in "Referenzwerte für die Nährstoffzufuhr", 2. Auflage, Umschau Braus Verlag, Frankfurt am Main, 2015, herausgegeben von der Deutschen Gesellschaft für Ernährung. Die Summe aus Wirkstoffkomponente und Formulierungsgrundlage beträgt in der Regel 100 Gew.-%.

Beispiele geeigneter Formulierungen zur Nahrungsergänzung sind Kapseln, Tabletten, Pillen, Pulverbeutel, Flüssigampullen und Fläschchen mit Tropfeinsätzen, im Übrigen die nachfolgend genannten Arzneiformen:
Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, halbfeste Arzneiformen, wie Salben, Cremes, Hydrogele oder Pasten, sowie flüssige Arzneiformen, wie Lösungen, Emulsionen, insbesondere Öl-in-Wasser-Emulsionen, Suspensionen, beispielsweise Lotionen. Auch implantierte Abgabevorrichtungen können zur Verabreichung erfindungsgemäßer Wirkstoffe verwendet werden. Ferner können auch Liposomen oder Mikrosphären zur Anwendung kommen.

Lebensmitteltechnische Formulierungen werden bevorzugt in Form von Kleinkindnahrung, Frühstücks-Zubereitungen, vor allem in Form von Müslis oder Riegeln, Sportlerdrinks, Komplettmahlzeiten, insbesondere im Rahmen von total bilanzierten Diäten, diätetische Zubereitungen, wie Diätdrinks, Diätmahlzeiten und Diätriegel, angeboten. Die Formulierungen werden vorzugsweise auf oralem Weg verabreicht.

Bei der Herstellung der Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z. B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z. B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Die obige Beschreibung bezog sich exemplarisch auf eine Senkung des LDL/HDL-Verhältnisses. Die Regulation des Lipidstoffwechsels kann sich jedoch auch auf eine Erhöhung des HDL-Wertes, oder auf eine Senkung des Gesamtcholesterinwertes, des LDL-Wertes, des Triglyceridwertes oder sowohl des Cholesterin- wie des Triglyceridwertes äußern, je nach Ausgangssituation: Im Allgemeinen bedeutet die Regulation hauptsächlich eine Normalisierung auffälliger Werte (nach Maßgabe ihres vorherigen Abweichens von Referenzwerten), wohingegen unauffällige Werte weniger beeinflusst werden. Neben diesen Veränderungen können, zusätzlich oder auch alternativ, der Kreatinin- und/oder der Blutzuckerwert günstig beeinflusst werden.

## Patentansprüche

1. Pharmazeutische Zusammensetzung auf Basis von Vitaminen zur Anwendung in der therapeutischen Regulierung des Lipidstoffwechsels, wobei die Vitamine wenigstens Folsäure, Vitamin B6 und Vitamin B12 beziehungsweise physiologisch akzeptable Derivate und/oder Salze davon beinhalten, **dadurch gekennzeichnet, dass** die Tagesdosis mindestens 0,2 mg Folsäure bzw. mindestens 4 mg Vitamin B6 bzw. mindestens 8 µg Vitamin B12, und höchstens 1,2 mg Folsäure beträgt, und wobei die physiologisch akzeptablen Derivate und/oder Salze folgendes beinhalten:
Amide und Ester der Folsäure, und Säure- und Basenadditionssalze sowie Mischformen der Folsäure gemäß Formel Ib: 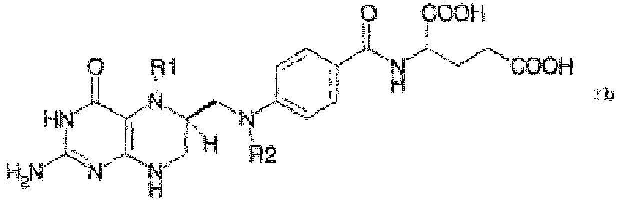 wobei R1 für Wasserstoff, Methyl, -HC=O oder -HC=NH steht und R2 für Wasserstoff oder -HC=O steht, oder R1 und R2 zusammengenommen eine Methylen- oder Methenylbrücke bilden;
Pyridoxal und Pyridoxamin sowie Ester des Pyridoxins, Pyridoxals und Pyridoxamins und deren Säureadditionssalze gemäß Formel IIa: 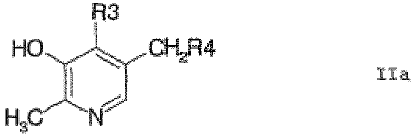 wobei R3 für CH₂OH, CHO oder CH₂NH₂ steht und R4 für OH oder OPO₃H₂ steht;
bzw. Aquocobalamin, Hydroxocobalamin, Nitrosocobalamin, Methylcobalamin und Adenosylcobalamin und deren Säureadditionssalze.

2. Pharmazeutische Zusammensetzung zur Anwendung in der Regulierung des Lipidstoffwechsels nach Anspruch 1, wobei die Tagesdosis höchstens 500 mg, bevorzugt mehr als 10 mg und höchstens 100 mg, oder ebenfalls bevorzugt höchstens 25 mg Vitamin B6 enthält.

3. Pharmazeutische Zusammensetzung zur Anwendung in der Regulierung des Lipidstoffwechsels nach Anspruch 1 oder 2, wobei die Tagesdosis an Folsäure mindestens 0,5 mg, und höchstens 1,1 mg, insbesondere höchstens 0,8 mg beträgt.

4. Pharmazeutische Zusammensetzung zur Anwendung in der Regulierung des Lipidstoffwechsels nach einem der Ansprüche 1 bis 3, wobei die Tagesdosis an Vitamin B12 mindestens 0,1 mg, und höchstens 4 mg, insbesondere höchstens 0,8 mg beträgt.

5. Pharmazeutische Zusammensetzung zur Anwendung in der Regulierung des Lipidstoffwechsels nach einem der Ansprüche 1 bis 4, wobei das Verhältnis der Tagesdosis an Folsäure zur Tagesdosis an Vitamin B12 mindestens 0,2, insbesondere mindestens 0,8 beträgt.

6. Pharmazeutische Zusammensetzung zur Anwendung in der Regulierung des Lipidstoffwechsels nach einem der Ansprüche 1 bis 5, ferner enthaltend Cholin, wobei die Tagesdosis an mit der Zusammensetzung verabreichtem Cholin mindestens 10 mg, insbesondere 90 bis 700 mg beträgt.

7. Pharmazeutische Zusammensetzung zur Anwendung in der Regulierung des Lipidstoffwechsels nach einem der Ansprüche 1 bis 6, ferner enthaltend Vitamin D3, wobei die Tagesdosis an mit der Zusammensetzung verabreichtem Vitamin D3 vorzugsweise mindestens 5 µg, insbesondere 10 bis 70 µg beträgt.

8. Pharmazeutische Zusammensetzung zur Anwendung in der Regulierung des Lipidstoffwechsels nach einem der Ansprüche 1 bis 7, ferner enthaltend Artischockenextrakt, wobei die Tagesdosis an mit der Zusammensetzung verabreichtem Artischockenextrakt mindestens 10 mg, insbesondere 40 bis 700 mg beträgt.

9. Pharmazeutische Zusammensetzung zur Anwendung in der Regulierung des Lipidstoffwechsels nach einem der Ansprüche 1 bis 8, ferner enthaltend mindestens 0,5 mg Riboflavin und/oder mindestens 5 mg Niacin.

10. Pharmazeutische Zusammensetzung zur Anwendung in der Regulierung des Lipidstoffwechsels nach einem der Ansprüche 1 bis 9, ferner enthaltend Carotinoide, bevorzugt Xanthophylle, weiter bevorzugt Astaxanthin, in einer Tagesdosis von mindestens 0,1 mg.

11. Pharmazeutische Zusammensetzung zur Anwendung in der Regulierung des Lipidstoffwechsels nach einem der Ansprüche 1 bis 10, ferner enthaltend Coenzym Q₁₀, in einer Tagesdosis von vorzugsweise mindestens 10 mg, insbesondere 50 bis 200 mg.

12. Pharmazeutische Zusammensetzung zur Anwendung in der Regulierung des Lipidstoffwechsels nach einem der Ansprüche 1 bis 11, ferner enthaltend Betain in einer Tagesdosis von mindestens 5 mg.

13. Pharmazeutische Zusammensetzung zur Anwendung in der Regulierung des Lipidstoffwechsels nach einem der Ansprüche 1 bis 12, ferner enthaltend eine oder mehrere Omega-3-Fettsäuren in einer Tagesdosis von, ggf. zusammengenommen, mindestens 20 mg.

14. Pharmazeutische Zusammensetzung zur Anwendung in der Regulierung des Lipidstoffwechsels nach einem der Ansprüche 1 bis 13, zur Verabreichung an Personen ohne Hyperhomocysteinämie.

15. Pharmazeutische Zusammensetzung zur Anwendung in der Regulierung des Lipidstoffwechsels nach einem der Ansprüche 1 bis 14, zur Verabreichung an Personen mit erhöhtem Blutzuckerwert oder mit erhöhtem Kreatininwert.

## Claims

1. A pharmaceutical composition on the basis of vitamins for use in the therapeutic regulation of lipid metabolism, wherein the vitamins include at least folic acid, vitamin B6 and vitamin B12 or physiologically acceptable derivates and/or salts thereof, respectively, **characterised in that** the daily dose includes at least 0.2 mg folic acid, a least 4 mg vitamin B6 and at least 8 µg vitamin B12, respectively, and at most 1.2 mg folic acid, and wherein the physiologically acceptable derivates and/or salts include the following:
amides and esters of folic acid, and acid and base addition salts as well as mixed forms of folic acid according to formula Ib: 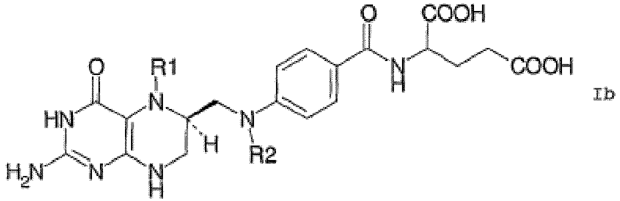 wherein R1 represents hydrogen, methyl, -HC=O or -HC=NH and R2 represents hydrogen or -HC=O, or R1 and R2 taken together form a methylen or methenyl bridge;
pyridoxal and pyridoxamine as well as esters of pyridoxin, pyridoxal and pyridoxamine and acid addition salts thereof according to formula IIa: 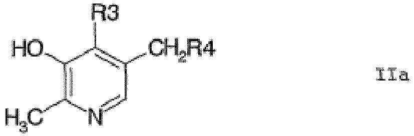 wherein R3 represents CH₂OH, CHO or CH₂NH₂ and R4 represents OH or OPO₃H₂;
and aquocobalamin, hydroxocobalamin, nitrosocobalamin, methylcobalamin and adenosylcobalamin and acid addition salts thereof, respectively.

2. The pharmaceutical composition for use in the regulation of lipid metabolism of claim 1, wherein the daily dose of vitamin B6 includes at most 500 mg, preferably more than 10 mg and at most 100 mg, or also preferably at most 25 mg.

3. The pharmaceutical composition for use in the regulation of lipid metabolism of claim 1 or 2, wherein the daily dose of folic acid is at least 0.5 mg, and at most 1.1 mg, in particular is at most 0.8 mg.

4. The pharmaceutical composition for use in the regulation of lipid metabolism of one of claims 1 to 3, wherein the daily dose of vitamin B12 is at least 0.1 mg, and at most 4 mg, in particular at most 0.8 mg.

5. The pharmaceutical composition for use in the regulation of lipid metabolism of one of claims 1 to 4, wherein the ratio of the daily dose of folic acid to the daily dose of vitamin B12 is at least 0.2, in particular at least 0.8.

6. The pharmaceutical composition for use in the regulation of lipid metabolism of one of claims 1 to 5, further including choline, wherein the daily dose of choline administered with the composition is at least 10 mg, in particular 90 to 700 mg.

7. The pharmaceutical composition for use in the regulation of lipid metabolism of one of claims 1 to 6, further including vitamin D3, wherein the daily dose of vitamin D3 administered with the composition is preferably at least 5 µg, in particular 10 to 70 µg.

8. The pharmaceutical composition for use in the regulation of lipid metabolism of one of claims 1 to 7, further including artichoke extract, wherein the daily dose of artichoke extract administered with the composition is at least 10 mg, in particular 40 to 700 mg.

9. The pharmaceutical composition for use in the regulation of lipid metabolism of one of claims 1 to 8, further including at least 0.5 mg riboflavin and/or at least 5 mg niacin.

10. The pharmaceutical composition for use in the regulation of lipid metabolism of one of claims 1 to 9, further including carotenoids, preferably xanthophylls, more preferably astaxanthin, in a daily dose of at least 0.1 mg.

11. The pharmaceutical composition for use in the regulation of lipid metabolism of one of claims 1 to 10, further including coenzyme Q₁₀, in a daily dose of preferably at least 10 mg, in particular 50 to 200 mg.

12. The pharmaceutical composition for use in the regulation of lipid metabolism of one of claims 1 to 11, further including betaine in a daily dose of at least 5 mg.

13. The pharmaceutical composition for use in the regulation of lipid metabolism of one of claims 1 to 12, further including one or more omega-3 fatty acids in a daily dose of, if applicable in total, at least 20 mg.

14. The pharmaceutical composition for use in the regulation of lipid metabolism of one of claims 1 to 13, for administration to persons without hyperhomocysteinemia.

15. The pharmaceutical composition for use in the regulation of lipid metabolism of one of claims 1 to 14, for administration to persons with an elevated blood glucose level or with an elevated creatinine level.

## Revendications

1. Composition pharmaceutique à base de vitamines pour une utilisation dans la régulation thérapeutique du métabolisme des lipides, dans laquelle les vitamines contiennent au moins de l'acide folique, de la vitamine B6 et de la vitamine B12 ou des dérivés physiologiquement acceptables et/ou des sels de ceux-ci, **caractérisée en ce que** la dose quotidienne est d'au moins 0,2 g d'acide folique ou au moins 4 mg de vitamine B6 ou d'au moins 8µg de vitamine B12 et au maximum de 1,2 mg d'acide folique, et dans laquelle les dérivés et/ou sels physiologiquement acceptables contiennent :
des amides et des esters de l'acide folique, et des sels d'addition d'acide ou de base ainsi que des formes mixtes de l'acide folique selon la formule Ib : 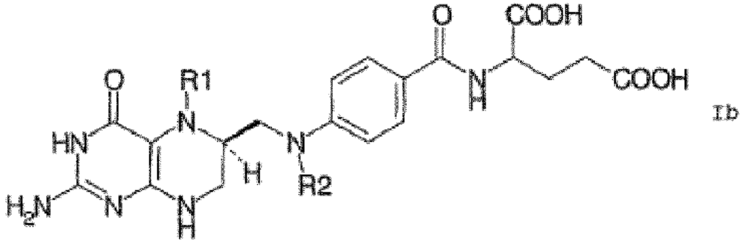 dans laquelle R1 représente de l'hydrogène, du méthyle, -HC=O ou -HC=NH et R2 représente de l'hydrogène ou -HC=O, ou R1 et R2 forment conjointement un pont de méthylène ou de méthényle ;
du pyridoxale et de la pyridoxamine ainsi que des esters de pyridoxine, de pyridoxale et de pyridoxamine et leurs sels d'addition d'acide selon la formule IIa : 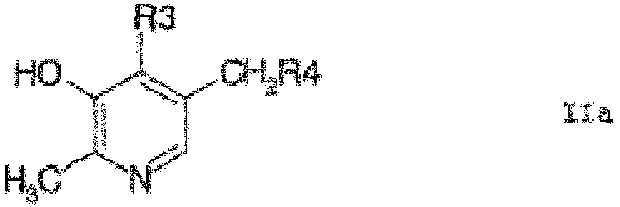 dans laquelle R3 représente CH₂OH, CHO ou CH₂NH₂ et R4 représente OH ou OPO₃H₂;
ou de l'aquocobalamine, de l'hydroxocobalamine, de la nitrosocobalamine, de la méthylcobalamine et de l'adénosylcobalamine et leurs sels d'addition d'acide.

2. Composition pharmaceutique pour une utilisation dans la régulation du métabolisme des lipides selon la revendication 1, dans laquelle la dose quotidienne contient au maximum 500 mg, de manière préférée plus de 10 mg et au maximum 100 mg, ou également de manière préférée au maximum 25 mg de vitamine B6.

3. Composition pharmaceutique pour une utilisation dans la régulation du métabolisme des lipides selon la revendication 1 ou 2, dans laquelle la dose quotidienne en acide folique est d'au moins 0,5 mg et au maximum de 1,1 mg, en particulier au maximum de 0,8 mg.

4. Composition pharmaceutique pour une utilisation dans la régulation du métabolisme des lipides selon l'une des revendications 1 à 3, dans laquelle la dose quotidienne en vitamine B12 est d'au moins 0,1 mg et au maximum de 4 mg, en particulier au maximum de 0,8 mg.

5. Composition pharmaceutique pour une utilisation dans la régulation du métabolisme des lipides selon l'une des revendications 1 à 4, dans laquelle le rapport entre la dose quotidienne en acide folique et la dose quotidienne en vitamine B12 est d'au moins 0,2, en particulier d'au moins 0,8.

6. Composition pharmaceutique pour une utilisation dans la régulation du métabolisme des lipides selon l'une des revendications 1 à 5, contenant en outre de la choline, dans laquelle la dose quotidienne en choline administrée avec la composition est d'au moins 10 mg, en particulier de 90 à 700 mg.

7. Composition pharmaceutique pour une utilisation dans la régulation du métabolisme des lipides selon l'une des revendications 1 à 6, contenant en outre de la vitamine D3, dans laquelle la dose quotidienne en vitamine D3 administrée avec la composition est de préférence d'au moins 5 µg, en particulier de 10 à 70 µg.

8. Composition pharmaceutique pour une utilisation dans la régulation du métabolisme des lipides selon l'une des revendications 1 à 7, contenant en outre de l'extrait d'artichaut, dans laquelle la dose quotidienne en extrait d'artichaut administré avec la composition est d'au moins 10 mg, en particulier de 40 à 700 mg.

9. Composition pharmaceutique pour une utilisation dans la régulation du métabolisme des lipides selon l'une des revendications 1 à 8, contenant en outre au moins 0,5 mg de riboflavine et/ou au moins 5 mg de niacine.

10. Composition pharmaceutique pour une utilisation dans la régulation du métabolisme des lipides selon l'une des revendications 1 à 9, contenant en outre du caroténoïde, de manière préférée de la xanthophylle, de manière davantage préférée de l'astaxanthine, en une dose quotidienne d'au moins 0,1 mg.

11. Composition pharmaceutique pour une utilisation dans la régulation du métabolisme des lipides selon l'une des revendications 1 à 10, contenant en outre du coenzyme Q₁₀ en une dose quotidienne de préférence d'au moins 10 mg, en particulier de 50 à 200 mg.

12. Composition pharmaceutique pour une utilisation dans la régulation du métabolisme des lipides selon l'une des revendications 1 à 11, contenant en outre de la bétaïne en une dose quotidienne d'au moins 5 mg.

13. Composition pharmaceutique pour une utilisation dans la régulation du métabolisme des lipides selon l'une des revendications 1 à 12, contenant en outre un ou plusieurs acides gras oméga-3 en une dose quotidienne, éventuellement conjointement, d'au moins 20 mg.

14. Composition pharmaceutique pour une utilisation dans la régulation du métabolisme des lipides selon l'une des revendications 1 à 13, pour une administration à des personnes sans hyperhomocystéinémie.

15. Composition pharmaceutique pour une utilisation dans la régulation du métabolisme des lipides selon l'une des revendications 1 à 14, pour une administration à des personnes présentant un taux de glycémie élevé ou un taux de créatinine élevé.
